(19) Europäisches Patentamt
European Patent Office
Office européen des brevets

(11) EP 0 822 859 B1

(12) **EUROPEAN PATENT SPECIFICATION**

(45) Date of publication and mention of the grant of the patent: **07.04.2004 Bulletin 2004/15**

(21) Application number: **96913893.2**

(22) Date of filing: **26.04.1996**

(51) Int Cl.[7]: **B01F 17/42**, B01J 13/00, C11D 1/18, C11D 1/52, C11D 1/58, C11D 1/72, D06M 13/148, D06M 13/352, D06M 13/402, D06M 13/46

(86) International application number: **PCT/US1996/006107**

(87) International publication number: **WO 1996/033800 (31.10.1996 Gazette 1996/48)**

(54) **COMPOSITIONS CONTAINING DIOL**

DIOL ENTHALTENDE ZUSAMMENSETZUNGEN

COMPOSITIONS CONTENANT DU DIOL

(84) Designated Contracting States:
**AT BE CH DE DK ES FI FR GB IE IT LI NL SE**

(30) Priority: **27.04.1995 US 430528**
**27.04.1995 US 430516**

(43) Date of publication of application:
**11.02.1998 Bulletin 1998/07**

(73) Proprietor: **Goldschmidt Chemical Corporation**
**Hopewell, Virginia 23860-1299 (US)**

(72) Inventor: **KEYS, Robert, O.**
**Columbus, OH 43221 (US)**

(74) Representative: **Körber, Wolfhart, Dr. rer.nat. et al**
**Patentanwälte**
**Mitscherlich & Partner,**
**Sonnenstrasse 33**
**80331 München (DE)**

(56) References cited:
**EP-A- 0 317 542**
**EP-A- 0 619 363**
**WO-A-94/18946**
**US-A- 3 779 934**
**US-A- 3 915 867**
**US-A- 3 992 319**
**US-A- 4 338 212**
**US-A- 4 692 277**
**US-A- 4 769 159**
**US-A- 4 873 079**
**US-A- 5 202 050**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 822 859 B1

Printed by Jouve, 75001 PARIS (FR)

## Description

## BACKGROUND OF THE INVENTION

The present invention relates to aqueous compositions containing solubilized or dispersed therein one or more cationic, anionic, amphoteric or nonionic surfactants which exhibit low solubility (or no solubility at all) in water, especially in the presence of electrolytes and/or pH agents. For purposes of this application, a substance is considered to be "solubilized" in water if the material is dissolved in the water or if it is uniformly dispersed or distributed therein, or emulsified therein, so as to exhibit the physical appearance and physical properties of a single-phase system (whether as an emulsion, an organic-based formula, or a water-based formula).

As is well known, surfactants can be used to perform a wide variety of useful purposes, ranging from cleaning and surface protection through deposition of coatings, fabric softening, foam stabilization, oil recovery, ore flotation, asphalt emulsification, achieving or enhancing rewetting effectiveness and penetrating power, and a large variety of other capabilities set forth hereinbelow. However, in many cases the ability to take advantage of such surfactants' useful properties is limited by the low solubility and/or dispersibility of many surfactants and surfactant blends in water.

There is also need in the marketplace for products with higher levels of concentration of surfactants or active ingredients, thus minimizing the amounts of water in the products. As the amount of water in the formulations is reduced, and as formulations add more (and more complex) ingredients, the fluidity and stability become.more difficult to maintain. Often the surfactants become insoluble gels when diluted in water, or become hazy or even split into different phases. Surfactants often become insoluble in formulations where the concentration of inorganic salts is very high. In other surfactant formulations, maintaining the fluidity and dispersibility of the surfactant in water are serious problems which limit their use and application. Many surfactants are difficult to even disperse in water, requiring both hot water and long periods of mixing for dissolution into solution.

The present invention relates in particular to liquid formulations containing one or more cationic compounds, such as: liquid fabric softeners of the type conventionally employed in the rinse cycle of automatic clothes washing machines; liquid textile softeners used for fabric finishing; compositions used in the paper industry for debonding and softening of paper fibers; hair and skin conditioners; compositions applied to clay-based products such as drilling muds to make them hydrophobic; and many other uses.

The present invention relates more particularly to novel compositions for liquid cationic formulations, wherein the ingredients of the composition contribute significantly to the ease of formulation, stability, dispersibility, fluidity and the performance properties of the compositions.

Cationics have achieved widespread usage because of their ability to impart to fabric, (i.e. articles of clothing, textiles, and so forth), paper, hair, and many other substrates, properties including softness to the touch, ease of handling, increased lubricity, and a reduced tendency to carry or pick up static electricity. One form in which cationics are provided is as a liquid, for instance as an emulsion or as a solution/suspension of the desired components. An appropriate controlled amount of the liquid cationic formulation is employed (poured into the washing machine or textile bath in which the fabric is being washed or rinsed; or applied to the hair; or added to the head tank of the paper making machine, or otherwise depending on the application).

Typically, in the case of liquid fabric softeners it is provided during the rinse cycle of the washing machine, either poured in by hand or metered in by an appropriate automatic metering device with which the washing machine is equipped. In the same vein, cationics (typically dialkyl quaternaries) are used in textile mills to add lubricity and finishing to the fabric prior to shipping the textile to market. The mill applies the cationic formulation in dilute emulsions and rapidly dries the excess water from the fabric. The fibers are thus lubricated and given a surface finish. Hair conditioners are applied as dilute cationic emulsions to the hair following its wash. Adding these conditioners (typically dialkyl quaternaries) reduces the tendency for tangling, improves the manageability, and imparts a soft feel to the hair strands. In the papermaking process, cationics called debonders are generally quaternary salt emulsions in water. These are added to the head tank wherein the dilute fibers are conditioned with the debonders just prior to being fed onto the papermaking screen. These debonders give improved softness feeling to the paper fibers. In all cases the cationics are added to hot water to make an emulsion, and then added to the substrate in water or added to the substrate in water or added as a high solids concentrate to the substrate, to impart softness, lubricity, antistatic properties, ease of handling of the substrate and to improve surface appearance.

It is believed that the user finds it to be desirable that the liquid cationic formulation is in the form of a moderately viscous fluid, rather. more viscous than water yet still capable of flowing under its own weight. Thus, for instance, having a formulation that at solids concentrations of less than 5% exhibit viscosities greater than 100 cps which would be effective in softening and disperse readily in cold water, such as the present invention, would be desirable in the marketplace. In other cases, the industrial user may want less viscous, fluid emulsions or concentrates that disperse easily, with fine particle sizes.

In the case of fabric softeners, formulations which would be low melting (compared to many softener raw

materials which must be heated to 90-120°F.) and are easily dispersed in room temperature water would save time and money in both equipment and production costs.

High solids formulations (or "ultras") which have solids contents greater than 20% have seen large commercial success over the last five years. The drive to increase solids contents, and to reduce handling and transportation costs is becoming ever more important. The desire ultimately to form a clear, highly active, high performance product when the product is dispersed in water is becoming an important objective. The standard emulsion type fabric softener ultras in the market suffer from thickening problems following production, causing reduced dispersibility in the rinse cycle.

There is a need for cationic formulations, including fabric softeners, which are nonflammable yet easy to handle and disperse in room temperature water. Most quaternary formulations contain isopropanol or ethanol as solvents in order to aid in production and handling. However, volatile solvents such as these are becoming an important environmental issue in states including California and Florida. Thus, a different technique for achieving fluidity and good dispersiblity, while avoiding the use of volatile solvents, is needed. Also, as interest grows in dilutable concentrated product which can be diluted by the customer (e.g. by 3-10 times) to make a regular (2-10%) concentration of the product as used, the need for making such products that are easily dispersible without resort to volatile or flammable solvents is very important.

Thus, there is still a need in this field for liquid cationic formulations which can be prepared more readily without encountering difficulties such as those described above, and are more concentrated and disperse easily in cold water. There is also a need in this field for cationic formulations which can be manufactured as concentrates, wherein formulators can produce consumer and industrial products easily, quickly and effectively with minimal equipment and heating requirements. There is also a need for products (especially for use in the textile and paper areas) which are not flammable but which avoid the handling and viscosity problems posed by the conventional less flammable substitutes such as propylene glycol, diethylene glycol and the like.

Hydrotropes or, more generally, coupling agents are added to surfactant formulations to increase the amount of the relatively water-insoluble surfactants that can be solubilized into the system. In most cases, they do not act as surfactants to lower surface tension but they often allow surfactants in the presence of salts or electrolytes to be added and subsequently dispersed into water at higher concentrations or at lower viscosities of the formulation than is otherwise achieved using only surfactant and water. These coupling agents assist surfactants by increasing the surfactant's solubility in water and its stability in the formulation, especially in the presence of salts, electrolytes and/or pH agents.

Hydrotropes or coupling agents generally contain short chained ($C_2$-$C_6$) hydrophobes with more bulky hydrophilic group(s) such as hydroxyl and/or sulfonates making them completely water-soluble. They are normally added to stabilize formulations of surfactants, salts and water and to hold them in single-phase systems.

Materials that have been proposed for use as coupling agents include hexylene glycol, propylene glycol, dipropylene glycol, diethylene glycol, any of various lower alkoxy-capped glycols or polyglycols, particularly where the glycol is ethylene glycol or propylene glycol, such as ethylene glycol monobutyl ether, alcohols such as isopropanol and ethanol, and certain aryl sulfonates such as sodium naphthalene sulfonate and sodium xylene sulfonate, as well as some phosphate esters. However, despite the abilities of these water-soluble products as coupling agents that have been suggested for these materials, there remains a need and an interest in identifying coupling agents and systems containing such coupling agents which not only exhibit superior stability and superior ability to solubilize relatively water-insoluble agents but also improve formulation fluidity, dispersiblity and product performance.

In addition, coupling agents that have improved permissible-exposure limits, higher flash points (over, for instance, isopropanol and ethanol), and lower odor (compared to, for example, butyl cellosolve or isopropanol) would have substantial importance to formulations and consumers. Other coupling agents such as sodium xylene sulfonate containing aromatic rings have come under environmental scrutiny in recent times.

The prior art concerning various cationic compositions is extensive, yet has not taught or suggested the considerable and unexpected benefits that are provided by the formulations which correspond to the present invention. For instance, U.S. Patent No. 5,399,272 discloses a clear or translucent liquid fabric softening composition containing any of certain ester-quaternary cationic compounds. The disclosure also requires any of certain alcohols, glycols, esters or ethers as solvent.

However, the disclosure of U.S. Patent No. 5,399,272 also requires a second quaternary compound and/or an amine oxide to serve as a dispersibility aid. The requirement for this dispersibility aid serves as a teaching that the disclosed solvent system does not adequately provide needed dispersiblity on its own, that is, in the absence of a dispersibility aid. This teaching thus serves to confirm the present state of the art, namely, that there remains a need for formulations which serve to solubilize and disperse cationic active agents without needing to resort to the addition of dispersibliity aids. After all, extra dispersibliity aids will add to the cost of materials, and having to add another cationic to the formulation could in some cases interfere with obtaining desired fluidity, maintaining a monophasic state, or obtaining the desired performance properties.

The composition disclosed in U.S. Patent No. 4,692,277 represents an attempt to incorporate certain diol solvents into hard surface cleaning formulations. The disclosure, however, is limited to liquid hard surface detergents/cleaners which contain at most 10% of a surfactant, and which must contain 1% to 30% of a detergency builder salt. The necessity of these components in the indicated amounts attests to the specific, limited nature of the teachings of this patent. More fundamentally, the disclosure of this patent was concerned solely with the solvent power of $C_6$-$C_{16}$ diols as to their effect on soap scum removal when combined with both surfactants and salt builders, and completely fails to suggest or appreciate that it is possible, through selection of components according to the present invention, to employ certain diols so as to attain the solubilization of much higher amounts of less soluble surfactant(s) while retaining the desired monophasic state of the resulting composition. Thus, in turn, this patent thereby also fails to suggest any of the many end-use (especially monophasic) formulations that can be prepared embodying the compositions afforded by the present invention in combination with other hydrophobic surfactants, be they active ingredients or otherwise.

EP-A 0 619 363 discloses aqueous liquid detergent compositions comprising from about 1 to 30% by weight of one or more surface active detergent compounds from among anionic, nonionic, cationic and amphoteric detergents and an insect repellent material. 2-ethyl-1,3-hexanediol and 2-(n-butyl)-2-ethyl-1,3-propanediol are named as suitable insect repellents.

US 3,915,867 discloses self-emulsifying domestic laundry fabric softener agents consisting of a mixture comprised of N-alkyl-N,N-di-(β-$C_{14}$-$C_{18}$-acyloxyethyl)-N-β-hydroxyethylammonium compounds and a solvent (inert vehicle) selected from a $C_1$-$C_4$ aliphatic alcohol or a $C_1$-$C_4$ aliphatic glycol. The mixture preferably contains about 75% by weight of the ammonium compound.

The present invention satisfies the needs identified above, and provides as well additional advantages that will become apparent in the following description.

## BRIEF SUMMARY OF THE INVENTION

One aspect of the present invention comprises homogeneous aqueous liquid compositions comprising (a) 2,2,4-trimethyl-1,3-pentane diol or a mixture of 2,2,4-trimethyl-1,3-pentane diol and 2-ethylhexane-1,3-diol; and (b) one or more cationic agents; and (c) water wherein said component (b) comprises at least 25 wt.% of said composition. In many embodiments, the component (b) comprises one or more cationic agents, i.e., quaternary ammonium compounds and/or amine salts as described herein.

Another aspect of the present invention comprises highly concentrated homogeneous compositions having the aforementioned composition, which concentrates are easily dispersible in water.

## DETAILED DESCRIPTION OF THE INVENTION

The compounds (a), sometimes referred to herein as diols, contribute essentially to many of the advantageous properties of the compositions of the present invention and are in many instances commercially available.

Preferred examples of compounds (a) include 2,2,4-trimethyl-1,3-pentane diol (referred to herein as "TMPD") or mixtures thereof with 2-ethylhexane-1, 3-diol.

The present discovery has particular utility with cationic surfactants that are either insoluble in water, or exhibit partial solubility in water such as up to 10 grams per 100 milliliters of water (in the absence of surfactants, coupling agents, or other solubility-enhancing additives). However, the present discovery also is useful with surfactants which exhibit even complete solubilities in water but which are difficult to disperse or to produce fluid, low viscosity formulations in water, and/or highly concentrated surfactant blends with or without electrolytes, builders and/or pH agents or other active agents.

Satisfactory cationic surfactants useful herein can readily be identified in well-known sources such as McCutcheon's Detergents & Emulsifiers, and the CTFA Cosmetic Ingredient Dictionary.

The cationic component useful in the present invention is one compound, or a combination of more than one compound, which compound or combination exhibits or imparts to the final product the properties desired for the intended use. Those properties include imparting to fabric, textiles, paper fibers, hair and other substrates a feeling of increased softness to the touch and a reduced tendency to carry or pick up static electricity.

Compounds one or more of which make up the cationic component, are typically nitrogenous compounds, e.g. secondary or tertiary amines, quaternary ammonium compounds, amine salts and diamine and triamine counterparts thereof.

As indicated, the present invention and its attendant advantages are realized with any cationic agent and particularly those which are mono- or di-(long chain alkyl) derivatives. Without intending to limit the scope of this invention, the following are provided as examples of cationic agents that can be employed in the present invention. That is, the present invention is intended to extend to compositions containing any other cationic compound that may not be mentioned herein.

Cationic agents usable in the present invention include, but are not limited to, nitrogenous compounds selected

from the group consisting of quaternized or acid salt derivatives of:

(i) alkylenediamines including compounds of the formula:

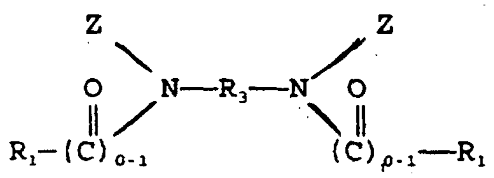

wherein each $R_1$ is an acyclic alkyl or alkylene $C_{12}$-$C_{21}$ hydrocarbon group, each Z is - $(R_2O)_{0-4}H$, or $-R_2H$, and $R_2$ and $R_3$ are divalent $C_1$-$C_6$ alkylene groups;

(ii) substituted imidazoline compounds having the formula:

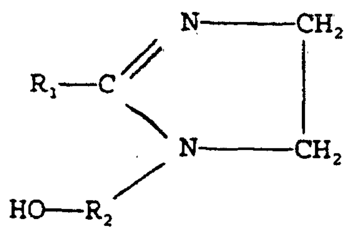

(iii) substituted imidazoline compounds having the formula:

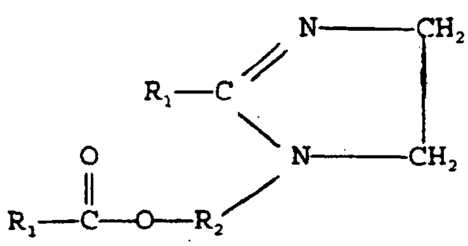

wherein $R_1$ and $R_2$ are defined as above;

(iv) reaction products of higher fatty acids with alkylenetriamines in, e.g., a molecular ratio of about 2:1, said reaction products containing compounds of the formula:

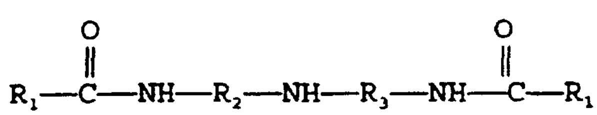

wherein $R_1$, $R_2$ and $R_3$ are def ined as above;

(v) substituted imidazoline compounds having the formula:

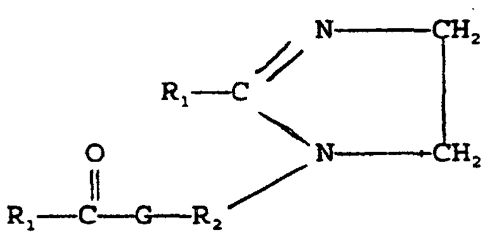

wherein G is -O- or -NH- and $R_1$ and $R_2$ are defined as above; and mixtures thereof.

Preferred examples of compounds of formula (i) are those derived from hydrogenated tallow fatty acids and the hydroxyalkylalkylenediamine N-2-hydroxyethylethylenediamine, such that $R_1$ is an aliphatic $C_{15}$-$C_{21}$ hydrocarbon group, and $R_2$ and $R_3$ are divalent ethylene groups.

A preferred example of compounds of formula (ii) is stearic hydroxyethyl imidazoline wherein $R_1$ is an aliphatic $C_{21}$ hydrocarbon group and $R_2$ is a divalent ethylene group.

A preferred example of compounds of formula (iv) is N,N"-ditallowalkanoyldiethylenetriamine where $R_1$ is an aliphatic $C_{15}$-$C_{21}$ hydrocarbon group and $R_2$ and $R_3$ are divalent ethylene groups.

A preferred example of compounds of formula (v) is 1-tallowamidoethyl-2-tallowimidazoline wherein $R_1$ is an aliphatic $C_{15}$-$C_{21}$ hydrocarbon group and $R_2$ is a divalent ethylene group.

Both N,N"-ditallowalkanoyldiethylenetriamine and 1-tallowethylamido-2-tallowimidazoline are reaction products of tallow fatty acids and diethylenetriamine, and are precursors of the cationic fabric softening agent methyl-1-tallowamidoethyl-2-tallowimidazolinium methylsulfate (see "Cationic Surface Active Agents as Fabric Softeners," R. R. Egan, Journal of the American Oil & Chemicals Society, January 1978, pages 118-121). N,N"-ditallowalkanoyldiethylenetriamine and 1-tallowamidoethyl-2-tallowimidazoline can be obtained from Witco Corporation. Methyl-1-tallowamidoethyl-2-tallowimidazolinium methylsulfate is sold by Witco Corporation under the trade name Varisoft® 475.

Other useful softening agents include cationic nitrogenous quaternary ammonium compounds and salts. In the cationic nitrogenous salts herein, the anion $A^{\ominus}$ provides electrical neutrality. Most often, the anion used to provide electrical neutrality in these salts is a halide, such as chloride, bromide, or iodide. However, other anions can be used, such as methylsulfate, ethylsulfate, acetate, formate, sulfate, carbonate, and the like. Chloride and methylsulfate are preferred herein as the anion $A^-$.

One type of cationic compounds are those containing one long chain acyclic aliphatic $C_8$-$C_{22}$ hydrocarbon group, selected from the group consisting of:

(vi) acyclic quaternary ammonium salts having the formula:

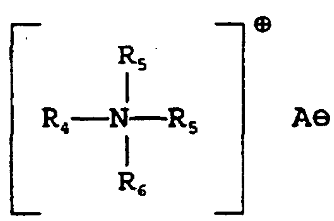

wherein $R_4$ is an acyclic aliphatic $C_8$-$C_{22}$ hydrocarbon group, alkyl, benzyl or ($C_4$-$C_{18}$ alkyl)-$(OCH_2CH_2)_{2\text{-}3}$-, $R_5$ and $R_6$ are $C_1$-$C_4$-saturated alkyl or hydroxyalkyl groups and $A^{\ominus}$ is an anion;

(vii) substituted imidazolinium salts having the formula:

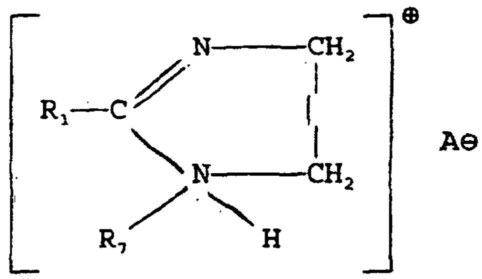

wherein $R_1$ is an acyclic alkyl or alkylene $C_{12}$-$C_{21}$ hydrocarbon group, $R_7$ is hydrogen or a $C_1$-$C_4$ saturated alkyl or hydroxyalkyl group, and $A^{\ominus}$ is an anion;
(viii) substituted imidazolinium salts having the formula:

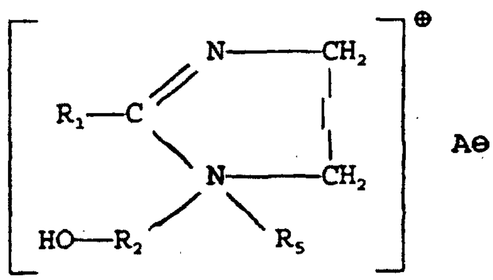

wherein $R_1$, $R_2$, $R_5$ and $A^{\ominus}$ are as defined above;
(ix) diquaternaries of the formula $(R^1\text{-}N(Z_2)\text{-}(CH_2)_{2\text{-}16}\text{-}N(Z_3))^{+2}\bullet 2A^-$
wherein $R_1$ and each Z are independently as defined above;
(x) alkylpyridinium salts having the formula:

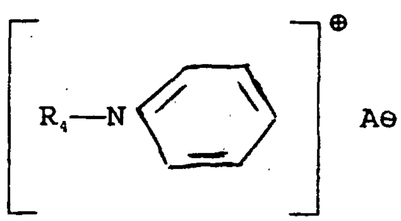

wherein $R_4$ is an acyclic aliphatic $C_8$-$C_{22}$ hydrocarbon group and $A^{\ominus}$ is an anion; and
(xi) alkanamide alkylene pyridinium salts having the formula:

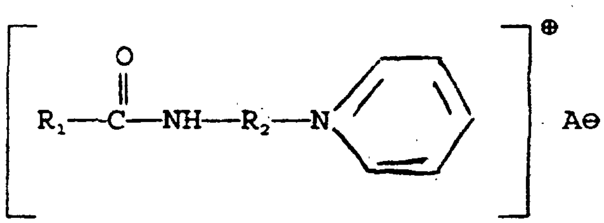

wherein $R_1$ is an acyclic aliphatic $C_{12}$-$C_{21}$ hydrocarbon group, $R_2$ is a divalent $C_1$-$C_6$ alkylene group, and $A^{\ominus}$ is

an anion; and mixtures thereof.

Examples of compound (vi) are the monoalkyltrimethylammonium salts such as monotallowtrimethylammonium chloride, mono(hydrogenated tallow)-trimethylammonium chloride, palmityltrimethylammonium chloride and soyatrimethylammonium chloride, sold by Witco Corporation under the trade names Adogen 471, Adogen 441, Adogen 444, and Adogen 415, respectively. In these compounds, $R_4$ is an acyclic aliphatic $C_{16}$-$C_{18}$ hydrocarbon group, and $R_5$ and $R_6$ are methyl groups. Mono(hydrogenated tallow)trimethylammonium chloride and monotallowtrimethylammonium chloride are preferred. Other examples of compound (vi) are behenyltrimethylammonium chloride wherein $R_4$ is a $C_{22}$ hydrocarbon group and sold under the trade name Kemamine® Q2803-C by Humko Chemical Division of Witco Corporation; soyadimethylethylammonium ethylsulfate wherein $R_4$ is a $C_{-16}$-$C_{18}$ hydrocarbon group, $R_5$ is a methyl group, $R_6$ is an ethyl group, and $A^-$ is an ethylsulfate anion; and methyl bis(2-hydroxyethyl)octadecylaminonium chloride wherein $R_4$ is a $C_{18}$ hydrocarbon group, $R_5$ is a 2-hydroxyethyl group and $R_6$ is a methyl group.

An example of compound (viii) is 1-ethyl-1-(2-hydroxyethyl)-2-isoheptadecylimidazolinium ethylsulfate wherein $R_1$ is a $C_{17}$ hydrocarbon group, $R_2$ is an ethylene group, $R_5$ is an ethyl group, and $A^-$ is an ethylsulfate anion.

Other fabric softening agents useful in the present invention include cationic nitrogenous salts having two or more long chain acyclic aliphatic $C_8$-$C_{22}$ hydrocarbon groups or one said group and an arylalkyl group. Examples include:

(xii) acyclic quaternary ammonium salts having the formula:

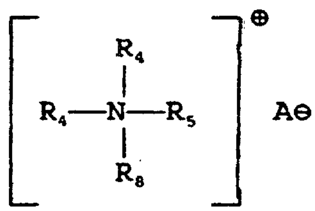

wherein each $R_4$ is an acyclic aliphatic $C_8$-$C_{22}$ hydrocarbon group, $R_5$ is a $C_1$-$C_4$ saturated alkyl or hydroxyalkyl group, $R_8$ is selected from the group consisting of $R_4$ and $R_5$ groups, and $A^{\ominus}$ is an anion defined as above;

(xiii) diamido quaternary ammonium salts having the formula:

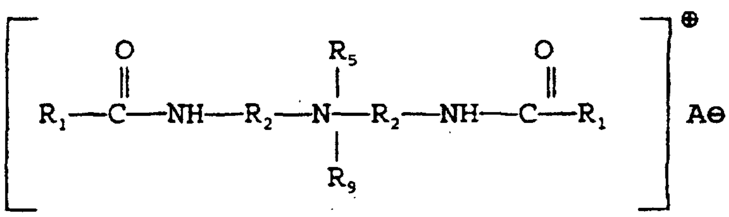

wherein each $R_1$ is an acyclic alkyl or alkylene $C_{12}$-$C_{21}$ hydrocarbon group, each $R_2$ is a divalent alkylene group having 1 to 3 carbon atoms, $R_5$ and $R_9$ are $C_1$-$C_4$ saturated alkyl or hydroxyalkyl groups, and $A^{\ominus}$ is an anion;

(xiv) alkoxylated diamido quaternary ammonium salts having the formula: ,

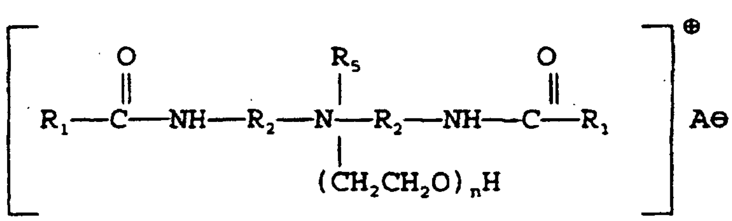

wherein n is equal to 1 to about 5, and $R_1$, $R_2$, $R_5$ and $A^{\ominus}$ are as defined above;
(xv) quaternary ammonium compounds having the formula:

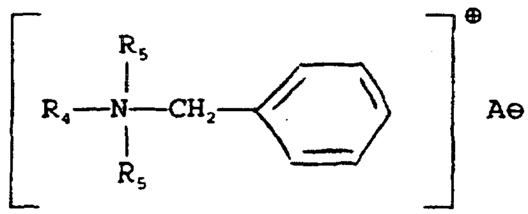

wherein each $R_4$ is an acyclic aliphatic $C_8$-$C_{22}$ hydrocarbon carbon group, each $R_5$ is a $C_1$-$C_4$ saturated alkyl or hydroxyalkyl group, and $A^{\ominus}$ is an anion;
(xvi) amide-substituted imidazolinium salts having the formula:

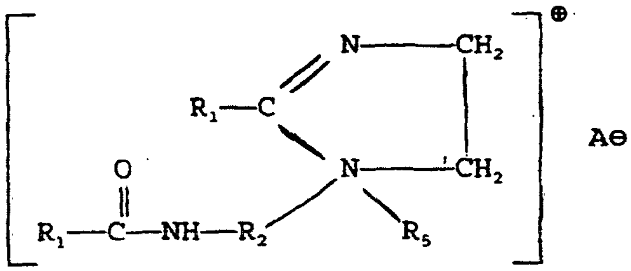

wherein each $R_1$ is an acyclic aliphatic $C_{12}$-$C_{21}$ hydrocarbon group, $R_2$ is a divalent alkylene group having 1 to 3 carbon atoms, and $R_5$ and $A^{\ominus}$ are as defined above or $R^5$ is -H; and
(xvii) ester-substituted imidazolinium salts having the formula:

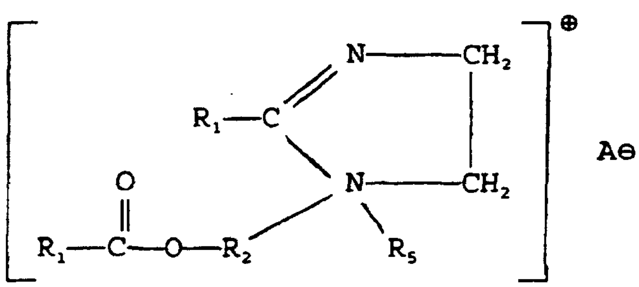

wherein $R_1$, $R_2$, $R_5$ and $A^{\ominus}$ are as defined above; and mixtures thereof.

Examples of compound (xii) are the well-known dialkyldimethylammonium salts such as ditallowdimethylammonium chloride, ditallowdimethylammonium methylsulfate, di(hydrogenated tallow)dimethylammonium chloride, distearyldimethylammonium chloride, dibehenyldimethylammonium chloride. Di(hydrogenated tallow)dimethylammonium chloride and ditallowdimethylammonium chloride are preferred. Examples of commercially available dialkyldimethylammonium salts usable in the present invention are di(hydrogenated tallow)dimethylammonium chloride (trade name Adogen 442), ditallowdimethylammonium chloride (trade name Adogen 470), distearyldimethylammonium chloride (trade name Arosurf TA-100), all available from Witco Corporation. Dibehenyldimethylammonium chloride wherein $R_4$ is an acyclic aliphatic $C_{22}$ hydrocarbon group is sold under the trade name Kemamine Q-2802C by Humko Chemical Division of Witco Corporation.

Examples of compound (xiii) are methylbis(tallowamidoethyl) (2-hydroxyethyl)ammonium methylsulfate and methylbis(hydrogenated tallowamidoethyl)(2-hydroxyethyl)ammonium methylsulfate wherein $R_1$ is an acyclic aliphatic

$C_{15}$-$C_{17}$ hydrocarbon group, $R_2$ is an ethylene group, $R_5$ is a methyl group, $R_9$ is a hydroxyalkyl group and $A^-$ is a methylsulfate anion; these materials are available from Witco Corporation under the trade names Varisoft 222 and Varisoft 110, respectively.

An example of compound (xv) is dimethylstearylbenzylammonium chloride wherein $R_4$ is an acyclic aliphatic $C_{18}$ hydrocarbon group, $R_5$ is a methyl group and $A^-$ is a chloride anion, which is sold under the trade name Varisoft SDC by Witco Corporation.

Examples of compound (xvi) are 1-methyl-1-tallowamidoethyl-2-tallowimidazolinium methylsulfate and 1-methyl-1-(hydrogenated tallowamidoethyl)-2-(hydrogenated tallow)imidazolinium methylsulfate wherein $R_1$ is an acyclic aliphatic $C_{15}$-$C_{17}$ hydrocarbon group, $R_2$ is an ethylene group, $R_5$ is a methyl group and $A^-$ is a chloride anion; they are sold under the trade names Varisoft 475 and Varisoft 445 respectively, by Witco.

Additional examples of fabric softening compounds useful in the present invention include

(xviii) compounds characterized by the formula:

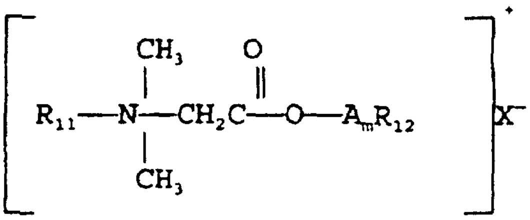

wherein

$R_{11}$ is a radical selected from the group consisting of (a) straight chain aliphatic hydrocarbon radicals each of which contains from 12 through 24 carbon atoms, (b) ether radicals each of which has the structure: $R_{13}O(CH_2O)_y$-, (c) amide radicals each of which has the structure:

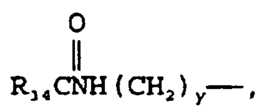

and (d) ester radicals each of which has the structure:

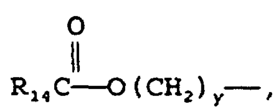

$R_{12}$ is a straight chain aliphatic hydrocarbon radical containing from 12 to 32 carbon atoms,
$R_{13}$ is a straight chain aliphatic hydrocarbon radical containing from 8 to 18 carbon atoms,
$R_{14}$ is a straight chain aliphatic hydrocarbon radical containing from 7 to 17 carbon atoms,
A is an alkoxy radical containing one oxygen atom and either two or three carbon atoms,
X is an atom selected from the group consisting of bromine and chlorine,
m is an integer of from 1 through 12, and
y is an integer which is either 2 or 3.

Yet additional examples of fabric softening compounds useful in the present invention include

(xix) compounds having the formula:

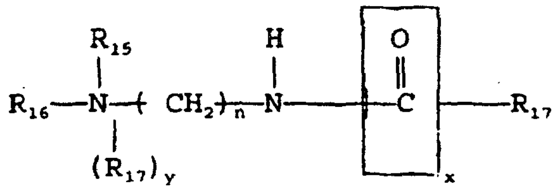

wherein

each $R_{15}$ is selected from the group consisting of hydrogen and $C_1$-$C_4$ alkyl, each $R_{16}$ is selected from the group consisting of $C_1$-$C_4$ alkyl and

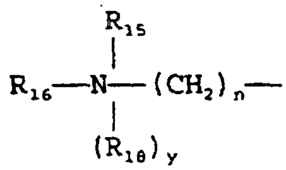

each $R_{17}$ is selected from the group consisting of $C_8$-$C_{28}$ alkyl and alkenyl groups, each $R_{18}$ is selected from the group consisting of hydrogen and $C_1$-$C_4$ alkyl, each y is 0 or 1, x is 0 or 1 and each n is from 1 to 6;

(xx) amides represented by the formula:

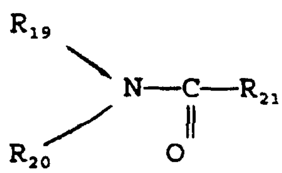

wherein

$R_{19}$ and $R_{20}$ are, selected independently, $C_{1-22}$ alk(en)yl aryl, or alkyl aryl groups, $R_{21}$ is hydrogen, or a $C_{1-22}$ alk(en)yl, aryl or alkyl-aryl group, or is O-$R_4$, wherein $R_{22}$ is a $C_{1-22}$ alk(en)yl, aryl or alkyl-aryl group, and $R_{21}$ and $R_{22}$ possibly containing 1 to 10 ethylene oxide units, or functional groups selected from hydroxy, amine, amide, ester, and ether groups; the aryl groups being possibly derived from hetero-cyclic compounds; at least one of the $R_{19}$ and $R_{20}$ groups contains 10 or more carbon atoms; the sum of carbon atoms in $R_{19}$+$R_{20}$+$R_{21}$ is equal to or greater than 14. Preferably, the sum of carbon atoms in $R_{19}$+$R_{20}$ is equal to or greater than 16.

Such species include N,N-ditallow acetamide, N,N-dicoconut acetamide, N,N-dioctadecyl propanamide, N-dodecyl, N-octadecyl acetamide, N-hexadecyl, N-dodecyl butanamide, N,N-ditallow benzamide, N,N-dicoconut benzamide, and N,N-ditallow 2-phenyl acetamide.

Additional fabric softening compounds useful in the present invention include all ester-quaternaries, including but not limited to:

(xxi) compounds of any of the formulas

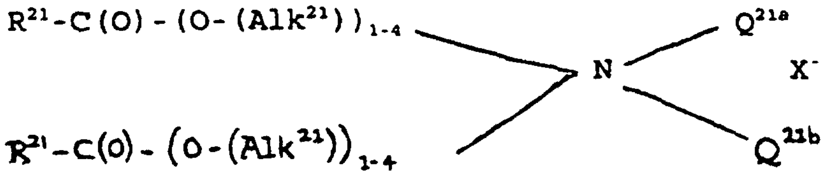

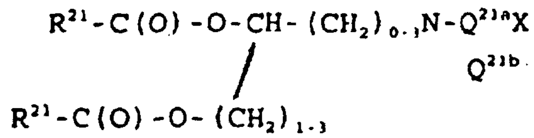

wherein

each $R^{21}$ is independently a saturated or unsaturated alkyl or alkylene radical containing 12 to 22 carbon atoms;
$Q^{21a}$ and $Q^{21b}$ are alkyl containing 1 to 4 carbon atoms or benzyl, $-CH_2CH_2OH$, or $-CH_2CH(OH)CH_3$, or $Q^{21a}$ can be $R^{21}-C(O)-(O-(Alk^{21}))_{1-4}-$;
each $Alk^{21}$ is independently $C_2H_4$, $C_1H_6$ or $C_4H_8$;
$R^2$ is alkyl containing 1 to 4 carbon atoms or benzyl, $-CH_2CH_2OH$ or $-CH_2CH(OH)CH_3$; and
$X^-$ is an anion;

(xxii) compounds of the formula

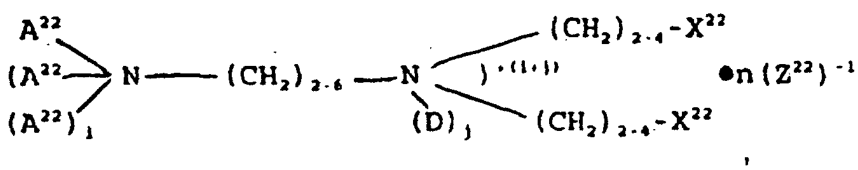

wherein

each $A^{22}$ is the same or different and each is alkyl containing up to 3 carbon atoms, benzyl, or $H-(Alk^{22}-O)_{1-3}-Alk^{22}-$ wherein each $Alk^{22}$ signifies $-CH_2CH_2-$, $-CH(CH_3)CH_2-$, or $-CH_2CH(CH_3)-$, provided further that one of the $A^{22}$ can be hydrogen;
D is methyl, ethyl, propyl, $-(CH_2)_{1-3}COO^-$, benzyl or hydrogen;
i is 0 or 1 and j is 0 or 1, provided that the sum of (i + j) is 1 or 2;
each $X^{22}$ is a straight or branched saturated or unsaturated aliphatic group containing up to 3 carbon-carbon double bonds and containing 11 to 23 carbon atoms;
n is (two minus the number of $-(CH_2)_{1-3}COO^-$ substituents present); and
$Z^{22}$ is an anion;

(xxiii) compounds of the formula

$$R^{23}-[C(O)O(CH_2)_{1-5}]_{0-1}-C(O)NH(CH_2)_{2-5}-N(R^{23a})(R^{23b})-$$

$$(CH_2)_{2-5}-OC(O)R^{23}X^-$$

wherein

each $R^{23}$ is independently straight or branched alkyl or alkenyl containing 8 to 22 carbon atoms;

$R^{23a}$ is straight or branched alkyl or hydroxyalkyl containing 1 to 3 carbon atoms, benzyl, or $-C_2H_4OC(O)R_4$ wherein $R^4$ is straight or branched alkyl or alkenyl containing 8 to 22 carbon atoms;
$R^{23b}$ is H, $-CH_3$, $-C_2H_5$ or benzyl; and
$X^-$ is an anion.

(In the foregoing, "Adogen", "Arosurf" and "Varisoft" are trademarks of Witco Corp.)

The compositions of the present invention also contain water but are diluted into or by water in each application area.

The amounts of the cationic, anionic, amphoteric or nonionic component, the one or more components corresponding to formula (1), and water, can vary within relatively large ranges, depending upon the degree of concentration of the components desired, and depending also on the particular characteristics of the particular components selected. The cationic component is present in an amount at least 25 wt% of the composition to afford the desired effect (i.e. fabric or textile softening, paper debonding, hair conditioning, and so forth, as the case may be) and can be present up to amounts on the order of 30 percent or higher, up to 60, 70, 80 or even 90 wt.% of the composition. These higher contents represent concentrates from which useful compositions can be formulated upon dilution or used as is, if desired due to their dispersibility in water.

Compositions in accordance with the present invention exhibit superior stability, by which is meant that they do not separate into more than one phase even upon standing, without agitation, for prolonged periods of time on the order of a year or longer. They generally form more fluid formulations, require lower levels of the diol or diol alkoxylate than of other coupling agents to function, and are nonflammable with high exposure limits. They also give very easy-to-disperse formulations even when the formulations are highly concentrated, and thus function better in each application. They generally give added fluidity to even difficult to handle surfactants and enable those same surfactants to be dispersed in much colder water than is the case with other coupling agents.

The compositions of the present invention are particularly useful in applications that take advantage of their ability to disperse hydrophobia material, to stabilize foam, and to enhance the penetration and wetting exhibited by the compositions. Examples of such applications include:

Oil dispersants and oil slick dispersants, wherein one applies onto oil (for instance, onto a film of oil) a sufficient amount of a composition according to the present invention, containing a sufficient concentration of surfactant, such that the composition disperses the oil.

Oil well stimulation and oil recovery aids, wherein one injects into an oil well a composition according to the present invention in order to penetrate into the surface of the borehole and assist liberation of crude oil from the matrix material into the hole, from which it can be brought to the surface.

Vehicles for hydrophobic sheeting agents such as mineral oil and silicone oil. Such oils can readily be dispersed in compositions, according to the present invention, and the resulting formulations are highly satisfactory when sprayed or otherwise applied to a surface (such as freshly washed automobile surfaces) to impart a lustrous, water-repellent film to the surface.

Formulation of fabric and textile softeners, wherein components capable of imparting fabric softening (typically, quaternary ammonium compounds such as di-($C_{12-22}$-alkyl)-di($C_1$-$C_4$ alkyl) ammonium chloride or methylsulfate, or 1,3-disubstituted imidazolinium salts) are incorporated into the composition thereby forming a fluid, monophasic, typically clear composition.

Paper deinking and ink flotation, wherein waste inked paper is pulped as a slurry in an aqueous liquid comprising a composition according to the present invention so that ink is liberated from the paper, and prevented from redepositing onto the paper; typically the ink is dispersed or even fully solubilized in the liquid composition of this invention or when the ink particles are floated from the fibers.

Paper debonding, wherein paper fibers are pulped in the headbox of a papermaking machine as a slurry in an aqueous liquid comprising a composition according to the present invention, just prior to feeding the slurry onto the dewatering screen, to improve the softness of the paper product formed.

Asphalt emulsions, wherein finely divided asphalt is emulsified (at loadings typically 1-20 wt.%), with or without particulate filler such as sand, in an aqueous phase which comprises the composition according to the present invention.

Hair and skin conditioning formulations, wherein effective amounts (e.g. 0.1 wt.% to 10 wt.% or more) of emollients, humectants, and/or slip and conditioning agents (e.g. organopolysiloxanes and the like) are incorporated with the composition of the present invention to create formulations that are monophasic and can be made to be translucent or even clear. Compounds suitable for use as emollients, humectants and conditioners in formulations for skin care or hair care can be found in the CTFA Cosmetic Ingredient Dictionary, 3d Edition, and in the CTFA Cosmetic Ingredient Handbook.

Corrosion inhibitors, wherein an effective amount of a hydrophobic corrosion inhibiting material (such as liquid or waxy-solid fatty ester, paraffinic hydrocarbon or silicone) is dispersed in a composition according to the present

invention. The resulting formulation is applied to any surface to which one desires to apply a film that protects against corrosion.

Ore flotation, wherein a relatively hydrophobic material used as e.g. a collector or frother --depending on the characteristics of the particular separation desired in the flotation cell-- is dispersed in a composition according to this invention. An amount effective to carry out the intended function is then fed (on a batch or continuous basis) to the cell. The composition of the present invention permits the formulator to improve the dispersibility of the collector(s) which are often very hydrophobic. This can often improve performance of the mineral separation by improving the efficiency of the chemical's dispersiblity. This can enable the operator to use smaller amounts (at the higher concentration of active now available) of the formulation to achieve the desired purpose.

Rinse aids, such as used in automatic dishwashers, wherein application of the composition of the present invention disperses residual hydrophobic matter, including cleaner residues and films.

Suspension concentrates and emulsifiable concentrates of herbicides, pesticides, miticides, fungicides, and/or bactericides, wherein one or more liquid or solid, generally hydrophobic, active ingredients are dispersed in a composition according to the present invention. The concentrate can be applied as is on or around desired vegetation; but is more often mixed (e.g. at the point of use) with water of dilution to form a final formulation having the desired concentration of active ingredient(s). This application takes advantage of the noteworthy property of this invention that addition of the water for dilution does not disrupt the monophasic state, nor the fluidity, of the formulation.

Generally speaking, the amount of coupling agent can range from about 0.1 wt.% or 10 wt.% to about 50 wt. %, with the particular amount readily identified by the formulator. Water may not necessarily be present, but usually is present in amounts that can be up to about 90-95 wt.%.

The one or more surfactants (which may exhibit low solubility in water) will generally be present in amounts on the order of at least 25 wt% , and similarly the particular amount can readily be ascertained by the formulator. The invention is particularly utilizable in embodiments wherein the amount of surfactant(s), in the aggregrate, exceeds 25 wt.%. Indeed, it has quite surprisingly been determined that compositions in accordance with the present invention can be prepared wherein the amount of surfactant(s) is at least 25 wt.%, or even 30 wt.% or higher, ranging up to 50 wt.% or higher, yet the composition remains monophasic and retains its fluidity and its ability to be compounded with other components without suffering phase separation, turbidity or excessive viscosity.

The compositions of the present invention can also optionally contain other components, depending on the additional properties one may wish to provide in the finished composition. Such additional components include, but are not limited to, additional coupling agents and solvents, thickeners, fragrances, coloring agents, hydrocarbon actives, and so forth.

The compositions of the present invention have particular usefulness in applications not calling for the presence of inorganic or organic salts. It is customary to incorporate quantities of such salts, known often as "builder" salts or "detergency builder" salts, particularly when cleaning functionality like hard surface cleaning is desired. However, the present invention is applicable to a considerable number of utilities that do not need the presence of builder salts, since they are not related to cleaning hard surfaces. The ability of the present invention to be so versatile and functional in applications without builder salts is one of the many unexpected and noteworthy aspects of the present invention.

FABRIC SOFTENERS

The component (a) is present in an amount sufficient to form with the cationic component a phase-stable, water-dispersible formulation. In general, satisfactory amounts of the one or more compounds (a) correspond to a weight ratio with respect to the amount of cationic component present of 1:30 to 5:1 (diol : fabric softener), and preferably 1:10 to 1:1.

The fabric softening compositions of the present invention can also contain conventional additives known to those familiar with this field, including colorants, fragrances, preservatives, and the like. In addition, if desired a small but effective amount up to about 2 wt.% of one or more inorganic salts, such as sodium chloride or calcium chloride, can be added to adjust the viscosity of the composition. Other components that can be present, and often are present, include monoalkyl nonionic materials such as fatty alcohols, fatty acid ethoxylates and propoxylates, monoalkyl esters or poly(ethylene glycol) esters of fatty acids, polysiloxanes, and amine-funcional polysiloxanes; other cationic surfactants; solvents such as short chain alcohols with up to about 6 carbon atoms (e.g. ethanol, isopropanol); lower glycols and glycol ethers, containing up to about 12 carbon atoms, such as ethylene glycol, diethylene glycol, propylene glycol, propylene glycol ether, propylene glycol butyl ether, and the like; polyethylene glycols; polypropylene glycols; fatty ethers; and hydrocarbons.

Compositions having the foregoing characteristics can readily be prepared by simply stirring together in appropriate equipment the diol component, with the one or more compounds constituting the cationic component, into the water, along with any other desired additives.

The cationic compositions of the present' invention afford a number of advantages not heretofore contemplated. One advantage is ease of formulation of these cationic compositions. Conventionally, emulsion-based cationic formulations can be made to a concentration of up to about 25 wt.% solids, employing high shear and requiring the addition of a salt such as calcium chloride for viscosity control. Solvent based (clear or transparent) cationic formulations can be made conventionally containing about 40 to about 60 wt.% solids, but often go through a gel-like phase which is very difficult to disperse, such that an acceptably uniform dispersion of the cationic component can be impossible to achieve. They normally require large levels (e.g. 10% or more) of flammable solvent such as isopropanol or ethanol, and/or hexylene glycol or propylene glycol, to formulate.

On the other hand, compositions prepared in accordance with the present invention exhibit a noticeable ease of dispersibility in water at any concentration level and can be thinned by adding $CaCl_2$ to form clear, fluid formulations. This is quite unique compared to those compositions outside the scope of the present invention requiring additions of e.g. isopropanol and/or ethanol and/or hexylene glycol, which revert back to the emulsion when salts are added. Much higher levels of alcohol and short-chained glycols are needed to maintain fluidity in such compositions. As much as 2 to 5 times more of such conventional solvents or coupling agents are needed for acceptable fluidity than is the case using the diols in accordance with the present invention. In addition, the compositions of the present invention do not readily gel when added to water for purposes of dilution. Thus, products of a concentration useful in the home can be prepared from concentrates very easily by simply dispersing an appropriate amount of the concentrate into room temperature water.

Another advantage is the appearance of the product. An opaque fabric softener or other cationic product is less desirable both because the appearance is considered to be unattractive to the consumer and also because it indicates that the distribution of the fabric softening components in the composition is not homogeneous. In the case of fabric softeners, this possibly results in uneven deposition of the fabric softener component on the clothing and possibly even results in staining of the fabric by the fabric softener component. On the other hand, fabric softener and other cationic compositions in accordance with the present invention can be made to appear clear or translucent, and upon addition of high amounts of water quickly form a correspondingly milky or clear, uniform product appearance. The iodine value (I.V.) of the cationic quaternaries to obtain clear formulations must be at least about 50 and is more preferably 60-90. Quaternaries derived from oleyl or soft tallow fatty acids especially can be made to give clear formulations.

The ease of formulation and dispersibility has other beneficial effects, including reduction in heating costs for formulators (who conventionally must heat the blend of components to help achieve the desired uniformity of distribution), and reduction in the amount of energy expended in mixing and transport. These features make it feasible to sell highly concentrated cationic formulations directly to the user, who prepares products having the concentrations conventionally employed from the concentrate by diluting an appropriate small quantity of concentrate with tap water or by adding it directly (for instance, by adding a small amount of a fabric softener concentrate) into the automatic dispenser on the machine where it is diluted and added to the rinse cycle.

Thus, both the emulsion type; clear and even clear gels can be made using the techniques disclosed herein. The diol (a) allows the cationic agent to be dispersed into water or to be diluted with water to any of a wide variety of concentrations and physical states (e.g. gels, clear products, and emulsions).

The following examples, which are intended for purposes of illustration and not intended to limit the scope of the protection sought for the invention described herein:

EXAMPLE 2

The following are examples of fabric softener formulations prepared in accordance with the present invention.

| FORMULATION B | |
|---|---|
| **Component** | **% by Weight** |
| Dihardtallowdimethylammoniumchloride, 75% solution in isopropanol ("Adogen 442", Witco Corp.) | 3.4 |
| 2,2,4-trimethyl-1,3-pentanediol | 1.1 |
| Deionized water | 95.5 |
| Total | 100.0 |

The quaternary ammonium compound was blended with the $C_8$ diol and this blend was added to the water at 32°C (90°F) with light agitation until the materials were completely dispersed. The resulting product was a cloudy, emulsion-type yet almost clear, liquid composition with a viscous appearance. This example demonstrates yet a further

advantage of the present invention, namely that employing the diols as described herein permit the preparation of a cationic composition having a given viscosity with the use of a smaller amount of cationic component than would be necessary to achieve that given viscosity level using the same cationic component, without using the diol.

The viscosity of Formulation B was about 0,175Pas (175 cps), whereas the viscosity of the "Adogen 442" alone, at the same level of quaternary in water, is about 0,015Pas (15 cps).

Among the advantages of the present invention is the high degree of dispersibility in water, even cold or room temperature water, and the resultant ability to formulate from a more highly concentrated form to any target concentration level in water (even room temperature) regardless of temperature with only minimal agitation. Other advantages include odor and low cost effectiveness compared to conventional coupling agents. The lack of formation of a gel phase during dilution or dispersion of the material in water is believed to be due to the material forming very fine particles when added to cold water; this feature also improves fabric softener performance in the washing machine, and provides freedom from having to add salts for adjustment of viscosity. Salts such as $CaCl_2$ may be added to reduce viscosity in those formulations where lower solids "clear" formulations are being produced. Additional advantages include the clarity of the final composition and freedom from having to include excess volatile organic components in the product.

Cationic emulsions are normally unstable, especially when subjected to freezing and thawing, and have shelf lives of only 3-5 months. On the other hand, emulsions utilizing diol exhibit much longer-term stabilities and better stability against freeze-thaw cycles. They also show good viscosity stability as well in dispersions up to about 10-15 wt.% in most cationic quaternary systems.

Additional Practical Exemplification

Laboratory work with compositions according to the present invention has demonstrated numerous specific advantageous aspects including, but not limited to, those set forth as follows.

The diols, particularly TMPD, reduce the melting point of the quaternary component; this feature greatly assists dispersibiliity of the cationic agent in water. Thus, for any given water temperature, the quaternary component can be dispersed more readily --and in a larger amount, if desired-- when one or more components (a) is present. The presence of the diol also enables water to be added to the quaternaries and cationics, as well as the customary mode of adding the material to the water. This is unprecedented, as normally most quaternaries will gel if water is added to them as the quaternary solids content go to below 40%.

The diols increase the viscosity of some formulations of quaternary compounds, easily by 10-fold or more. For a given content of quaternary compound(s), there is generally a range of the diol component within which optimum high viscosity is exhibited; higher amounts of diol with respect to the amount of quaternary present can reduce the viscosity compared to that obtained at lower diol levels, and thereby form easily dispersed formulations.

Softening Performance

It has been determined that cationic formulations containing the diol provide fabric softening performance which is superior to that provided by comparable formulations without the diol. Typical results are in the following table:

| Comparison of Softening Performance with TMPD vs. Emulsions w/o TMPD (All tests used quaternary as 0.1 wt.% of fabric weight.) | | | |
|---|---|---|---|
| Softener | Wt.% of active in deionized water | Ratio (wt.%) of Quat.: TMPD | Softening Rank (5=Best) |
| Di (hydrogenated tallow) dimethyl ammonium chloride | 3% | -- | 3.1 |
| (as 75% formulation in isopropanol) | 3% | 4:1 | 3.8 |
| (Adogen "442") | 3% | 3:2 | 4.1 |
| | | | (3=Best) |
| Methyl-1-tallow amidoethyl-2-tallowimidazoliniummethyl sulfate | 23.3% | -- | 1.3 |
| ("Varisoft 475-90") | 26.8% | 3:2 | 1.7 |

In these experiments, all quaternary concentrations were held exactly equivalent; and the experiments were performed using identical testing conditions. Thus, any performance improvement is attributed to the presence of the

TMPD.

These data confirm that the presence of the diol contributes additional fabric softening capability to the formulated product.

Clear Formulations

Formulations in accordance with this invention which exhibit acceptable clarity can be obtained with appropriate balance of the amounts of the softener and diol components, the amounts of other components such as electrolytes, and the conditions (especially temperature) under which the formulations are prepared. In general, at lower softener contents, a higher ratio of TMPD or other diol to the system needs to be observed.

Additional examples of formulations according to this invention are:

NONFLAMMABLE IMIDAZOLINIUM QUATERNARY FORMULATION (useful as e.g. fabric softener, textile finishing, paper debonder)

A. Concentrate

80% Methyl-1-tallow amidoethyl-2-tallow imidazolinium methyl sulfate ("Varisoft 475") (as 90% conc. in isopropanol)
15% TMPD
5% $H_2O$

This product exhibits the following beneficial properties:

- Dispersible in room temperature water
- Non-flammable
- Easy to handle
- No need to heat or store the product at elevated temperatures
- Stable dispersions at from 1-12% solids
- Superior softening and antistatic properties vs. softener without the diol
- Good rewetability
- Thick viscosity at about 5-8% solids

| Typical Properties | |
|---|---|
| Appearance | light yellow liquid |
| Total Solids(%) | 72% |
| Density (gm/cc) | .92 |
| Flash Point (PMCC) | >93°C (>200°F) |

B. Formulations for Use

| | | | |
|---|---|---|---|
| Wt.% of solids: | 3% | 5.5% | 8% |
| Approx. Viscosity (cps.) | 20 | 75 | 600 |
| % Quat: | 4.3% | 7.85% | 11.4% |
| % Tap Water | 95.7% | 92.15% | 88.6% |

Procedure for concentrate dilution:

Measure water required for dispersion into a suitable mixing vessel. Water temperature should be above 21°C (70°F). Add room temperature quaternary to the water with mild agitation. Continue agitation for 15 to 30 minutes until the softener is completely dispersed. Add dye, fragrance and preservative as required or needed. Solids of greater than 7% may need to be thinned using a $CaCl_2$ brine. Small amounts (less than .5% $CaCl_2$) should be used.

HIGH VISCOSITY/LOW-SOLIDS QUATERNARY FORMULATION

A. Concentrate

42.5% Methyl bis (tallow amidoethyl) 2-hydroxyethyl ammonium methyl sulfate ("Varisoft 222 LM-90") (as 90% conc. in isopropanol)
42.5% "Adogen 442" (as 75% conc. in isopropanol)
15% TMPD

This product exhibits the following beneficial properties:

- Good softening and antistatic control
- Easy to handle (fluid at 60°F.)
- Dispersible at water temperature as low as 65°F
- High viscosity at less than 4% solids
- Good formulation stability down to 1%
- Can achieve elevated viscosity when Quat is dispersed at water temperatures below 75°F.

| Typical Properties | |
|---|---|
| Appearance | light yellow liquid |
| Pour Point | 10°C (50°F) |
| Flash Point (PMCC) | 30°C (86°F) |
| % IPA | 10° |
| Total solids (%) | 70% |
| Solids Formulation Range (%) | .5 to 8% |

B. Typical End-Use Formulations

| Solids Content | 2.5% Solids | | 4% Solids | |
|---|---|---|---|---|
| Dispersion Water Temp. | >24°C (>75°F) | <24°C (<75°F) | >24°C (>75°F) | <24°C (<75°F) |
| Viscosity of Formulation, | 0,015 Pas (15 cps) | 0,15 Pas (150 cps) | 0,18 Pas (180 cps) | 0,40 Pas (400 cps) |

Formulation:

| | | |
|---|---|---|
| % Product | 3.6% | 5.7% |
| % Water | 96.4% | 94.3% |

Procedure:

Measure water at desired temperature (to achieve desired viscosity) into a vessel equipped with a mixing agitator. Add room temperature quaternary 21°-27°C (70-80°F) slowly to water under agitation. Mixing smoothly without whipping air into the dispersion reduces foam problems and having air contained in the thicker dispersion. Agitate until completely dispersed- usually 15-30 minutes depending on the type of agitation. Add dye, fragrances, preservatives as desired. If foam develops during mixing or bottling, add a few ounces of defoamer such as Antifoam B (Dow Corning).

"CLEAR" (ADJUSTABLE VISCOSITY) SOFTENER FORMULATION

A. Concentrate

80% Ditallow dimethyl ammonium chloride ("Adogen 470") (as 75% conc. in isopropanol)
20% TMPD

This product exhibits the following beneficial properties:

- Excellent softening with good antistatic control
- Clear formulation
- Variable solids content from 10 to 40% or greater
- Easy to handle
- Can be used as a "dilutable" to form a thick emulsion-type product when added to water down to 3-6% solids
- versatile viscosity from thin to thick using e.g. $CaCl_2$ as thinning agent
- Can be used to form cold-water-dispersible formulations of 4-6% solids (viscosity of 0,07-0,2 Pas (70-200cps))
- Great dispersibility in cold water

| Typical Properties | |
|---|---|
| Appearance | Clear yellow fluid |
| liquid | |
| Total solids (%) | 58% |
| Density (gm/cc) | 0.87 |
| Flash point (PMCC) | 21°C (70°F) |
| Min. Handling Temp. | 4°C(40°F) |
| Cloud Point | 7°C(45°F) |

B. Typical End-Use Formulations

| | 18-20% | 24-26% solids | 30-35% solids |
|---|---|---|---|
| Concentrate | 33% | 42% | 52% |
| $CaCl_2$ | .05-.15% | .2-.4% | .02-.125% |
| Water | Balance to 100% | | |
| Dye, Preservative and Fragrance | As desired or recommended by suppliers | | |

Procedure:

Charge water into a suitable mixing vessel. Add proper level of $CaCl_2$ to obtain stable, clear final softener formulation. Add all required Quat to water and begin to agitate. Upon mild agitation a "clear" fluid (or viscous if desired) softener formulation will form. Initially during blending, periods of hazy or even opaque dispersion may exist prior to "clearing." Too much $CaCl_2$ should be avoided as it will lead to splitting out of the softener. Too little $CaCl_2$ will result in high viscosity or even a gel. If too much $CaCl_2$ is added, water can be added diluting the formulation and switching the formulation back to a clear formulation.

| PAPER DEBONDERS | |
|---|---|
| Ingredient | Amount (wt.%) |
| | A |
| Di(hard tallow) dimethyl ammonium methyl sulfate | 50 |

(continued)

| PAPER DEBONDERS | |
|---|---|
| Ingredient | Amount (wt.%) |
| | A |
| TMPD | 20 |
| TMPD x 2-mole ethoxylate | 20 |
| Water | 5 |
| | (melts at 24-27°C (75-80°F); dispersible in 29°C (85°F) water) |

| NON-FLAMMABLE TEXTILE FINISHING FORMULATION | |
|---|---|
| Ingredient | Amount (wt.%) |
| Methyl bis (tallowamidoethyl) - 2-hydroxyethyl ammonium methylsulfate (as 85% conc. in hexylene glycol) | 81 |
| TMPD | 14 |
| Water | 5 |
| Approximate pour point = 13°C (55°F). | |
| Approximate minimum water dispersal temperature = 10°C (50°F). | |

The following are additional examples of more particular formulations embodying the compositions of the present invention. These examples are provided for purposed of illustration, and should not be deemed to limit the scope of the invention.

EXAMPLES

| Carwash Sheeting Spray | | |
|---|---|---|
| Component (wt.%) | Typical Amount wt.% | Exemplary Amount |
| Dicoco dimethyl ammonium chloride (78% in isopropanol) | 10-30 | 20 |
| Diol (e.g. TMPD) | 2-10 | 5 |
| Mineral seal oil | 20-30 | 25 |
| Water | 40-60 | 50 |

| Clear Fabric Softener | | |
|---|---|---|
| Component (wt.%) | Typical Amount (wt.%) | Exemplary Amount |
| Di (soft tallow) dimethyl ammonium chloride | 30-40 | 35 |
| TMPD | 5-12 | 10 |
| $CaCl_2$ | 0.1-0.4 | 0.2 |
| Fragrance/dye preservative | trace | trace |
| Water | 50-60 | 55 |

| Paper Debonder Concentrate | | |
|---|---|---|
| Component (wt.%) | Typical Amount (wt.%) | Exemplary Amount |
| Di (hard tallow) dimethyl ammonium methylsulfate | 40-50 | 45 |

(continued)

| Paper Debonder Concentrate | | |
|---|---|---|
| Component (wt.%) | Typical Amount (wt.%) | Exemplary Amount |
| TMPD | 20-30 | 25 |
| TMPD x 3-mole ethoxylate | 20-30 | 25 |
| Water | 2.5-10 | 5 |

| Textile Softener Concentrate (cold water dispersible, nonflammable) | | |
|---|---|---|
| Component (wt.%) | Typical Amount (wt.%) | Exemplary Amount |
| Methyl-1-tallow amidoethyl -2-tallow imidazolinium methylsulfate | 50-75 | 70 |
| TMPD | 10-15 | 14 |
| TMPD x 3-mole ethoxyate | 10-15 | 12 |
| Water | 2-10 | 4 |

## Claims

**1.** A homogenous liquid cationic composition comprising:

(a) 2,2,4-trimethyl-1,3-pentane diol or a mixture of 2,2,4-trimethyl-1,3-pentane diol and 2-ethylhexane-1,3-diol;
(b) one or more cationic agents; and
(c) water;

wherein said component (b) comprises at least 25 wt.% of said composition.

**2.** The composition in accordance with claim 1 wherein said one or more cationic agents includes di(hydrogenated tallow) dimethyl ammonium chloride.

**3.** The composition in accordance with claim 1 wherein said one or more cationic agents includes methyl bis(tallowamidoethyl-)-2-hydroxyethyl ammonium methylsulfate.

**4.** The composition in accordance with claim 1 wherein said one or more cationic agents includes methyl-1-tallowamidoethyl-2-tallow-imidazolinium methylsulfate.

**5.** A composition in accordance with claim 1 wherein said one or more cationic agents includes one or more compounds selected from the group consisting of compounds of any of the formulas:

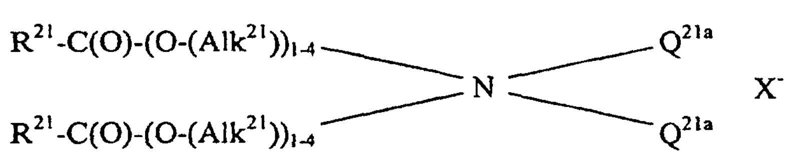

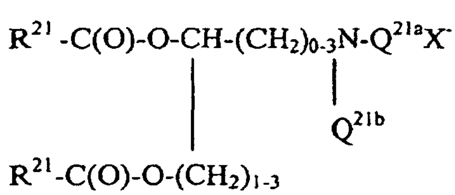

$$R^{23}\text{-}[C(O)O(CH_2)_{1\text{-}5}]_{0\text{-}1}\text{-}C(O)NH(CH_2)_{2\text{-}5}\text{-}N(R^{23a})(R^{23b})\text{-}$$

$$(CH_2)_{2\text{-}5}\text{-}OC(O)R^{23}X^-$$

wherein

each $R^{21}$ is independently a saturated or unsaturated alkyl or alkylene radical containing 12 to 22 carbon atoms;
$Q^{21a}$ and $Q^{21b}$ are alkyl containing 1 to 4 carbon atoms or benzyl, $-CH_2CH_2OH$, or $-CH_2CH(OH)CH_3$, or $Q^{21a}$ can be $R^{21}$-C(O)-(O-($Alk^{21}$))$_{1\text{-}4}$-;
each $Alk^{21}$ is independently $C_2H_4$, $C_3H_6$ or $C_4H_8$;
$R^2$ is alkyl containing 1 to 4 carbon atoms or benzyl, $-CH_2CH_2OH$ or $-CH_2CH(OH)CH_3$;
each $R^{23}$ is independently straight or branched alkyl or alkenyl containing 8 to 22 carbon atoms;
$R^{23a}$ is straight or branched alkyl or hydroxyalkyl containing 1 to 3 carbon atoms, benzyl, or $-C_2H_4OC(O)R^4$, wherein $R^4$ is straight or branched alkyl or alkenyl containing 8 to 22 carbon atoms;
$R^{23b}$ is H, $-CH_3$, $-C_2H_5$ or benzyl; and
$X^-$ is an anion.

**6.** A composition according to claim 1 wherein said component (b) comprises two or more cationic agents.

**7.** A composition according to claim 1 wherein said component (b) comprises at least 30 wt.% of said composition.

**8.** A composition according to claim 1 wherein said component (b) comprises at least 40 wt. % of said composition.

**9.** A composition according to claim 1 wherein said component (b) comprises at least 50 wt. % of said composition.

**Patentansprüche**

**1.** Homogene flüssige kationische Zusammensetzung, die

(a) 2,2,4-Trimethyl-1,3-pentandiol oder eine Mischung aus 2,2,4-Trimethyl-1,3-pentandiol und 2-Ethylhexan-1,3-diol;
(b) ein oder mehrere kationische Mittel und
(c) Wasser umfasst,

worin die Komponente (b) mindestens 25 Gew.-% der Zusammensetzung umfasst.

**2.** Zusammensetzung nach Anspruch 1, worin das eine oder die mehreren kationischen Mittel dihydriertes Talgdimethylammoniumchlorid umfasst(en).

**3.** Zusammensetzung nach Anspruch 1, worin das eine oder die mehreren kationischen Mittel Methylbis(talgamidoethyl-2)-2-hydroxyethylammoniummethylsulfat umfasst (umfassen).

**4.** Zusammensetzung nach Anspruch 1, worin das eine oder die mehreren kationischen Mittel Methyl-1-talgamidoethyl-2-talgimidazoliniummethylsulfat umfasst (en).

**5.** Zusammensetzung nach Anspruch 1, worin das eine oder die mehreren kationischen Mittel eine oder mehrere Verbindungen umfassen, die aus der Gruppe gewählt sind, die aus Verbindungen einer der Formeln besteht:

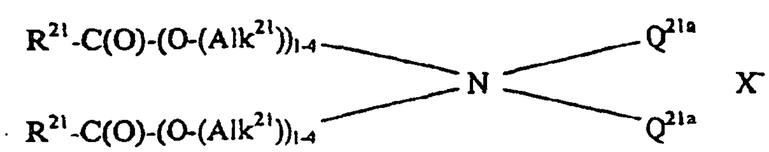

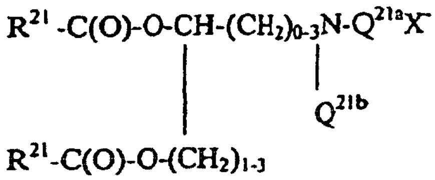

$$R^{23}\text{-}[C(O)O(CH_2)_{1\text{-}5}]_{0\text{-}1}\text{-}C(O)NH(CH_2)_{2\text{-}5}\text{-}N(R^{23a})(R^{23b})\text{-}$$
$$(CH_2)_{2\text{-}5}\text{-}OC(O)R^{23}X^-$$

worin

$R^{21}$ voneinander unabhängig eine gesättigte oder ungesättigte Alkyl- oder Alkylengruppe, die 12 bis 22 Kohlenstoffatome enthält, bedeutet;
$Q^{21a}$ und $Q^{21b}$ ein Alkyl, das 1 bis 4 Kohlenstoffatome enthält oder Benzyl, $-CH_2CH_2OH$ oder $-CH_2CH(OH)CH_3$ bedeuten oder $Q^{21a}$ $R^{21}$-C(O)-(O-($Alk^{21}$))$_{1\text{-}4}$- bedeuten kann;
$Alk^{21}$ voneinander unabhängig $C_2H_4$, $C_3H_6$ oder $C_4H_8$ bedeutet;
$R^2$ Alkyl, das 1 bis 4 Kohlenstoffatome enthält oder Benzyl, $-CH_2CH_2OH$ oder $-CH_2CH(OH)CH_3$ bedeutet;
$R^{23}$ voneinander unabhängig ein geradkettiges oder verzweigtes Alkyl oder Alkenyl, das 8 bis 2 Kohlenstoffatome enthält, bedeutet;
$R^{23a}$ ein geradkettiges oder verzweigtes Alkyl oder Hydroxyalkyl, das 1 bis 3 Kohlenstoffatome enthält, Benzyl oder $-C_2H_4OC(O)R^4$, worin $R^4$ ein geradkettiges oder verzweigtes Alkyl oder Alkenyl, das 8 bis 22 Kohlenstoffatome enthält, bedeutet;
$R^{23b}$ H, $-CH_3$, $-C_2H_5$ oder Benzyl bedeutet und
$X^-$ ein Anion bedeutet.

**6.** Zusammensetzung nach Anspruch 1, worin die Komponente (b) zwei oder mehrere kationische Mittel umfasst.

**7.** Zusammensetzung nach Anspruch 1, worin die Komponente (b) mindestens 30 Gew.-% der Zusammensetzung umfasst.

**8.** Zusammensetzung nach Anspruch 1, worin die Komponente (b) mindestens 40 Gew.-% der Zusammensetzung umfasst.

**9.** Zusammensetzung nach Anspruch 1, worin die Komponente (b) mindestens 50 Gew.-% der Zusammensetzung umasst.

## Revendications

**1.** Composition cationique liquide homogène comprenant :

(a) du 2,2,4-triméthyl-1,3-pentane diol ou mélange de 2,2,4-triméthyl-1,3-pentane diol et du 2-éthylhexane-1,3 diol ;
(b) un ou plusieurs agents cationiques ; et
(c) de l'eau ;

dans laquelle ledit composant (b) comprend au moins 25 % en poids de ladite composition.

**2.** Composition selon la revendication 1, dans laquelle ledit agent cationique ou lesdits agents cationiques comprennent du chlorure de di(suif hydrogéné) diméthyl ammonium.

**3.** Composition selon la revendication 1, dans laquelle ledit agent cationique ou lesdits agents cationiques comprennent du méthylsulfate de méthyl bis(suif-amidoéthyl)-2-hydroxyéthyl d'ammonium.

4. Composition selon la revendication 1, dans laquelle ledit agent cationique ou lesdits agents cationiques comprennent du méthylsulfate de méthyl-1-suif-amidoéthyl-2-suif-imidazolinium.

5. Composition selon la revendication 1, dans laquelle ledit agent cationique ou lesdits agents cationiques comprennent un plusieurs composés sélectionnés parmi le groupe regroupant des composés de l'une des formules suivantes :

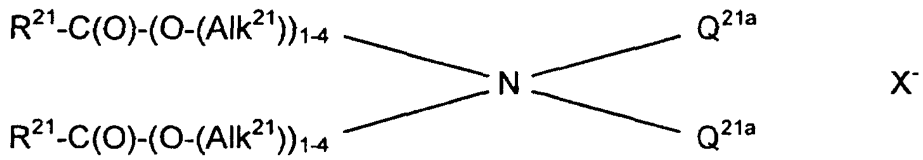

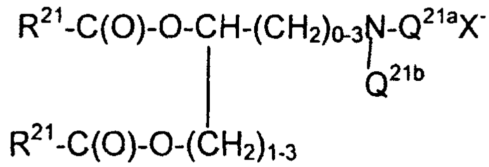

$$R^{23}\text{-}[C(O)O(CH_2)_{1\text{-}5}]_{0\text{-}1}\text{-}C(O)NH(CH_2)_{2\text{-}5}\text{-}N(R^{23a})(R^{23b})\text{-}(CH_2)_{2\text{-}5}\text{-}OC(O)R^{23}X^-$$

où

chaque $R^{21}$ est indépendamment un radical alkyle ou alkylène saturé ou insaturé contenant 12 à 22 atomes de carbone ;
$Q^{21a}$ et $Q^{21b}$ sont un alkyle contenant 1 à 4 atomes de carbone ou un benzyle, $-CH_2CH_2OH$ ou $CH_2CH(OH)CH_3$, ou $Q^{21a}$ peut être $R^{21}\text{-}C(O)\text{-}(O\text{-}(Alk^{21}))_{1\text{-}4}$
chaque $Alk^{21}$ est indépendamment $C_2H_4$, $C_3H_6$ ou $C_4H_8$ ;
$R^2$ est un alkyle contenant 1 à 4 atomes de carbone ou bien un benzyle, ou $-CH_2CH_2OH$ ou $-CH_2CH(OH)CH_3$ ;
chaque $R^{23}$ est indépendamment un alkyle ou un alkényle linéaire ou ramifié contenant 8 à 22 atomes de carbone ;
$R^{23a}$ est un alkyle ou hydroxyalkyle linéaire ou ramifié contenant 1 à 3 atomes de carbone, un benzyle ou $-C_2H_4OC(O)R^4$, dans lequel $R^4$ est un alkyle ou un alkényle linéaire ou ramifié contenant 8 à 22 atomes de carbone,
$R^{23b}$ est H, $-CH_3$, $-C_2H_5$ ou un benzyle ; et
$X^-$ est un anion.

6. Composition selon la revendication 1, dans laquelle ledit composant (b) comprend deux agents cationiques ou plus.

7. Composition selon la revendication 1, dans laquelle ledit composant (b) comprend au moins 30 % en poids de ladite composition.

8. Composition selon la revendication 1, dans laquelle ledit composant (b) comprend au moins 40 % en poids de ladite composition.

9. Composition selon la revendication 1, dans laquelle ledit composant (b) comprend au moins 50 % en poids de ladite composition.